# EUROPEAN PATENT APPLICATION

(11) **EP 1 696 230 A1**
(43) Date of publication of application: **30.08.2006**
(21) Application number: 04807281.3
(22) Date of filing: 17.12.2004
(51) Int. Cl.: G01N 27/62, G01N 33/68, C12Q 1/37

(54) **PROTEIN ANALYSIS METHOD**

(30) Priority: 17.12.2003 JP 2003419921
(71) Applicant: Yamauchi, Yoshio, Hino-shi Tokyo 191-0052 (JP); Arakawa, Takashi, Hachioji-shi Tokyo 192-0372 (JP); Isobe, Toshiaki, Hachioji-shi Tokyo 192-0916 (JP)
(72) Inventor: Yamauchi, Yoshio, Hino-shi Tokyo 191-0052 (JP); Arakawa, Takashi, Hachioji-shi Tokyo 192-0372 (JP); Isobe, Toshiaki, Hachioji-shi Tokyo 192-0916 (JP)
(74) Representative: Gudat, Axel
(86) International application number: PCT/JP2004/018923
(87) International publication number: WO 2005/059538

(57) **Abstract**

A method for analyzing proteins according to which two types of samples containing proteins are compared using a mass spectrometer, so that the proteins which are included in respective samples are identified and the mass ratio of a protein of the same type that is included in the respective samples is analyzed, wherein the method for analyzing proteins is **characterized by** including the steps of:
respectively digestting said two types of samples containing proteins at portions of a certain amino acid using a restriction enzyme so as to prepare samples containing peptides;
modifying peptides which are included in said respective samples containing peptides with labeling compounds having different masses due to isotopes, so that peptides of the same type that are included in the respective samples containing peptides have different masses;
mixing the samples containing peptides that have been respectively labeled with isotopes, separating and quantifying the mixed sample for each peptide and measuring the MS spectrum, and finding the content ratio of peptides of the same type having different masses due to isotope labeling;
selecting a peptide of which the amino acid sequence should be identified from among the peptides in reference to said MS spectrum and qualitatively analyzing the amino acid sequence of selected peptide from the mass spectrum of the product ions which are generated from the peptide;
identifying a corresponding protein from known-DNA sequences on the basis of the amino acid sequence of said peptide; and
finding the ratio of the content of said identified protein included in said samples containing respective proteins on the basis of the value obtained from separation quantification using the difference in the mass of said peptides that have been modified with isotopes.

## Description

### RELATED APPLICATIONS

This application claims priority to the Japanese Patent Application 2003-419921 dated on December 17, 2003 and is hereby incorporated with reference for all purposes.

### FIELD OF THE INVENTION

The present invention relates to a method for analyzing proteins, in particular, to an improvement on an analysis method using a mass spectrometer.

### BACKGROUND OF THE INVENTION

Gene function analysis has been progressing as the relationship between vast gene information that has been clarified as projects for analyzing gene information, such as the genome project in recent years, has progressed, and a variety of proteins which interact in a complex manner within cells has been clarified. Proteome analysis is an attempt to comprehensively understand the relationship between a varieties of proteins for supporting the functions of cells. Current analysis technology, however, requires a large amount of time and effort for analyzing proteins, and therefore, a method for comprehensively and quickly understanding change in proteomes which are a group of proteins having such a variety.

In electrophoretic analysis which is generally carried out as a conventional separation analysis for proteins, though separation can be carried out with high separation power, there is a problem, such that automation is difficult and it is also difficult to secure reproducibility and quantification.

Therefore, in recent years, liquid chromatography, mass spectrometers and data analysis systems have been combined, and a large scale protein identification system for consistently and automatically carrying out a process from the separation of a sample to the identification of proteins has been developed.

Patent Document 1: Japanese Unexamined Patent Publication 2003-107066

### DISCLOSURE OF THE INVENTION

### PROBLEM TO BE SOLVED BY THE INVENTION

In addition, demand for finding change in the amount of cell proteins between a normal state and a sick state, as well as in the amount of protein which is manifested in tissue while being generated, sick tissue and tissue that has genetically mutated has become high. That is to say, quantitative information, such as the amount of proteins, is simultaneously required, in addition to identification to proteins within cells.

Therefore, comparison of quantity between samples using an ICAT™ (registered trademark) reagent has been widely carried out (see, for example, Patent Document 1). In this ICAT™ (registered trademark) method, however, there is a problem, such that the pre-process operation is complicated.

### MEANS FOR SOLVING PROBLEM

The present invention is provided in view of the above described problem, and an object thereof is to provide a method for analyzing proteins according to which identification of proteins and quantitative information thereon can be obtained in a simple process.

To accomplish the above object, a method for analyzing proteins in accordance with the present invention comprises, two types of samples containing proteins are compared using a mass spectrometer, so that the proteins which are included in respective samples are identified and the mass ratio of a protein of the same type that is included in the respective samples is analyzed, wherein the method for analyzing proteins is characterized by including the steps of:
respectively digesting said two types of samples containing proteins at portions of a certain amino acid using a restriction enzyme so as to prepare samples containing peptides;
modifying peptides which are included in said respective samples containing peptides with labeling compounds having different masses due to isotopes, so that peptides of the same type that are included in the respective samples containing peptides have different masses;
mixing the samples containing peptides that have been respectively labeled with isotopes, separating and quantifying the mixed sample for each peptide and measuring the MS spectrum, and finding the content ratio of peptides of the same type having different masses due to isotope labeling;
selecting a peptide of which the amino acid sequence should be identified from among the peptides in reference to said MS spectrum and qualitatively analyzing the amino acid sequence of selected peptide from the mass spectrum of the product ions which are generated from the peptide;
identifying a corresponding protein from known-DNA sequences on the basis of the amino acid sequence of said peptide; and
finding the ratio of the content of said identified protein included in said samples containing respective proteins on the basis of the value obtained from separation quantification using the difference in the mass of said peptides that have been modified with isotopes.

In the method for analyzing proteins of the present invention, it is preferable that O-methyl-isourea and its stable isotopes are used as said labeling compounds.

In the method for analyzing proteins of the present invention, it is preferable that in said step of finding the content ratio of peptides of the same type, when two peaks of peptides of the same type having different masses due to said modifying compounds in the MS spectrum are compared, the quantitative ratio is corrected by getting rid of the overlapping region with the peak of a peptide labeled with an naturally-occurring isotope.

### EFFECTS OF THE INVENTION

In accordance with a method for analyzing proteins according to the present invention, it becomes possible to obtain quantitative information on proteins in a simple process.

### BRIEF DESCRIPTION OF THE DRAWINGS

Fig 1 is a diagram showing a method for analyzing proteins according to an embodiment of the present invention; and
Figs 2(a) and 2(b) are diagrams showing data processing.

### DESCRIPTION OF THE PREFERRED EMBODIMENT

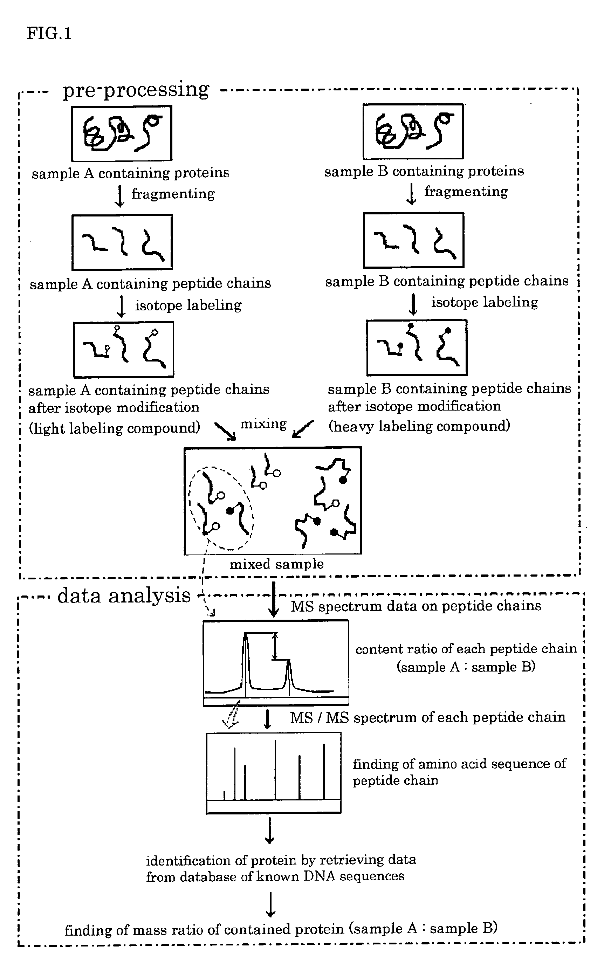

In the following, the preferred embodiments of the present invention are described in reference to the drawings. Fig 1 is a diagram showing the flow of a method for analyzing proteins according to the present embodiment. According to the method for analyzing proteins of the present embodiment, a tandem mass spectrometer is used, and two types of samples containing proteins are compared, so that proteins which are included in the respective samples are identified and the mass ratio of proteins of the same type which are included in respective samples is analyzed.

As for the two types of samples having proteins, one sample taken from tissue in a normal state and another sample taken from tissue in a sick state, for example, are used for tissue of the same type. Thus, quantitative comparison of expression level is carried out on the manifested protein component which is included in these samples containing proteins.

The process according to the method for analyzing proteins of the present embodiment can be roughly divided into the step of pre-processing samples (up to the stage where mixed samples have been prepared in Fig 1) and the step of analyzing data that has been obtained using a tandem mass spectrometer (the part of determining of the content ratio using an MS spectrum and identifying of proteins using an MS/MS spectrum and a database).

In the step of pre-processing samples, two types of samples containing proteins to be compared are processed. The main purpose here is to label the respective samples with a labeling compound that may have different mass numbers depending on the isotopes, so that proteins are labeled to show which sample they originate from on the basis of the mass difference. In addition, it is necessary to digest protein components into shorter peptides in order to determine the primary structure of proteins using a mass spectrometer.

Therefore, in two types of samples containing proteins (which are respectively referred to as sample A and sample B in Fig 1), protein components are first digest into peptides at portions of a certain amino acid using a restriction enzyme, and thus, samples A and B containing peptides are obtained from the original samples. Here, peptides indicate those of which the number of amino acids is in a range from several to in the tens. That is to say, peptides indicate those having a length that can be analyzed using a mass spectrometer.

Next, the respective samples containing peptides are modified with labeling compounds having different masses. As these labeling compounds, two compounds having different mass numbers where a portion of an element that forms a compound is replaced with another isotope are prepared. Fig 1 shows a case where peptide containing sample A is modified with a light labeling compound and peptide containing sample B is modified with a heavy labeling compound.

The respective samples containing peptides which have been labeled with isotopes in this manner are mixed.

Next, the thus obtained mixed sample is analyzed using liquid chromatography and a tandem mass spectrometer. In the present embodiment, the respective peptides in mixed sample are first separated by means of liquid chromatography.

Then, the respective peptides are introduced to a tandem mass spectrometer so that an MS spectrum is obtained in the first mass spectrometer and an MS/MS spectrum is obtained in the second mass spectrometer. The thus obtained data is analyzed as follows.

Some of the respective peptides originate from sample A and others originate from sample B, and these two types have a certain mass difference in mass resulting from isotopic labeling. Therefore, it can be seen in the above described MS spectrum data that the peak of the peptides originating from sample A and the peak of the peptides originating from sample B are at different points. The height of these respective peaks (or the peak area or the like) is compared, and thereby, the ratio of the content of this type of peptide in sample A to that in sample B can be found.

Next, MS/MS spectrum data on the above described respective peptides is analyzed in order to identify which protein the peptides are a part of. At this time, by reference to the above described MS spectrum, the type of peptide to identify the protein can be selected from among the measured peptides.

For the selected peptides, the amino acid sequence of each peptide can be determined from the MS/MS spectrum data in accordance with a known analysis technology. That is to say, on the basis of the amino acid sequence in a peptide, a gene and a protein which correspond to this peptide can be identified using a known database that stores known DNA sequences.

The ratio of the content of the peptide in sample A to that in sample B is found as in the above, and therefore, the ratio of the content of the protein that corresponds to this peptide in sample A to that in sample B can be found.

The outline of the present embodiment is described in the above. In the following, the respective steps are described in detail.

In the first step, two types of samples A and B containing proteins are respectively digest at portions of a certain amino acid using a restriction enzyme so that the proteins fragment into peptides. As this restriction enzyme, Lys-C/P is used, so that the proteins are digest on the C terminal side of lysine.

In the next step, the samples that have fragmented into peptides as described above are modified with labeling compounds having different masses, and thereby, peptides which are respectively included in samples A and B have different masses.

As the labeling compounds, O-methyl-isourea that can be represented by the following formulas (1) and (2) is used.

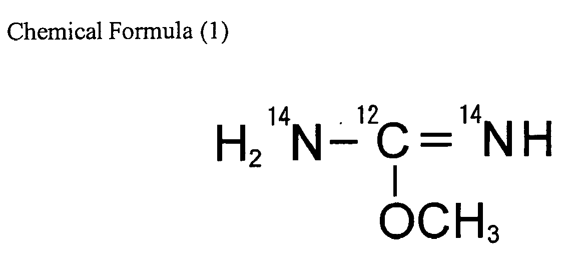

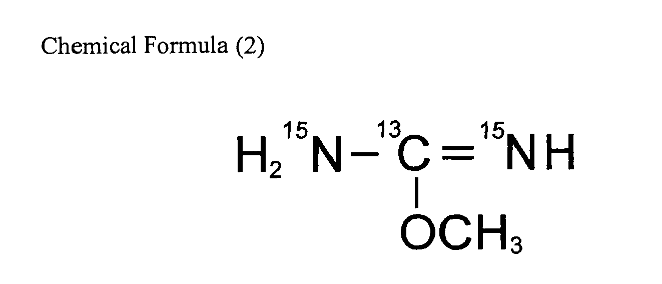

Here, the numbers at the top left of C and N in the above described chemical formulas represent mass numbers. That is to say, in the heavy labeling compound (chemical formula (2)), nitrogen atoms N having a mass number of 14 and carbon atoms C which are not in the methyl group and having a mass number of 12 in the light labeling compound (chemical formula (1)) are replaced with stable isotopes, that is, nitrogen atoms N having a mass number of 15 and carbon atoms C having a mass number of 14, respectively. Therefore, the heavy labeling compound (having a mass number of 45) and the light labeling compound (having a mass number of 42) have a difference in mass of 3Da.

The above described O-methyl-isourea combines with a portion of a lysine residue through the following reaction.

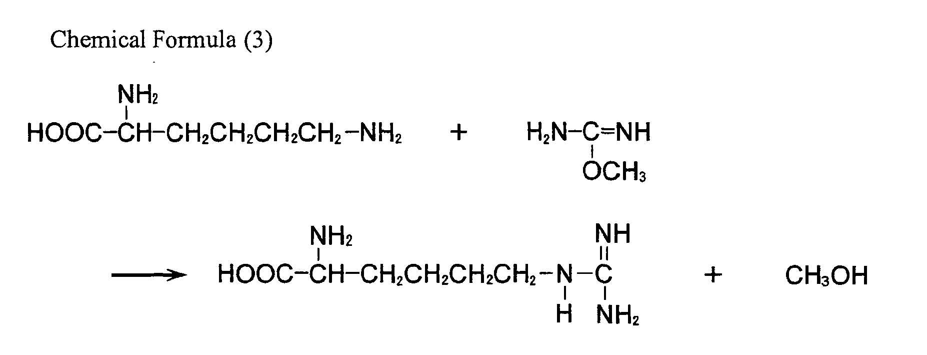

Thus, the peptides that are included in sample A are modified with a light reagent and the peptides that are included in sample B are modified with a heavy reagent, and the respective reagents have isotopes. After that, these samples A and B which have been labeled with isotopes are mixed.

Next, the above described mixed samples are separated by means of liquid chromatography (LC). There is no difference in the chemical properties between the heavy labeling compound and the light labeling compound, that is to say, there is no difference other than the mass number in peptides of the same type between those originating from sample A and those originating from sample B, and therefore, peptides of the same type originating from sample A and sample B have the same peak when separated by means of LC. The mixed sample is analyzed using a mass spectrometer after the separated by means of LC.

In the present embodiment, as the mass spectrometer, a quadrupole time-of-flight tandem mass spectrometer (MS/MS) is used, and the MS spectrum and MS/MS spectrum are measured. As for this device configuration, the same as that of the prior art can be used. In addition to this, it is possible to use a Fourier transform mass spectrometer (FT-MS). The peptides in the mixed sample that has been separated by means of LC are ionized through ESI (electrospray ionization) or the like and are fed to the first mass spectrometer. Certain precursor ions are selected from the above described ions in the first mass spectrometer and are fed to the second mass spectrometer. These precursor ions are irradiated with an argon gas or the like, and thus fragment into smaller product ions, which are then detected by the second mass spectrometer. As described above, the mass spectrum (MS/MS spectrum) of the product ions which have fragmented from the selected peptide ions is obtained. In addition, at the same time, the MS spectrum data for the peptides before fragmenting into product ions can also be obtained.

The thus obtained MS spectrum data and MS/MS spectrum data are stored in a computer, so that proteins which are included in the samples are identified through data processing in the following manner, and furthermore, the relative ratio of proteins included in the two samples is also found.

First, the mass ratio of each peptide originating from sample A to that originating from sample B is found from the MS spectrum data. That is to say, the peak of one peptide (originating from sample A) in the MS spectrum and the peak (originating from sample B) at a point at such a distance that the difference in mass is 3 vis-à-vis the formed peak are compared, and thereby, the relative ratio of the amount of certain peptides that is included in sample A to that included in sample B can be found.

Here, most natural elements have a stable isotope intrinsic to the element. Therefore, as for the molecular weight of any given compound, several peaks may exist, depending on how much isotope of what mass number each element that forms the compound includes. It is possible to find the ratio of the respective peaks from the ratio of the isotopes of the element that forms the compound in nature. Therefore, these peaks of the isotopes which exist in nature are taken into consideration, and the portions of the peaks resulting from stable isotopes in nature needs to be subtracted when the quantitative ratios of samples A and B containing protein that has been identified as described above are compared.

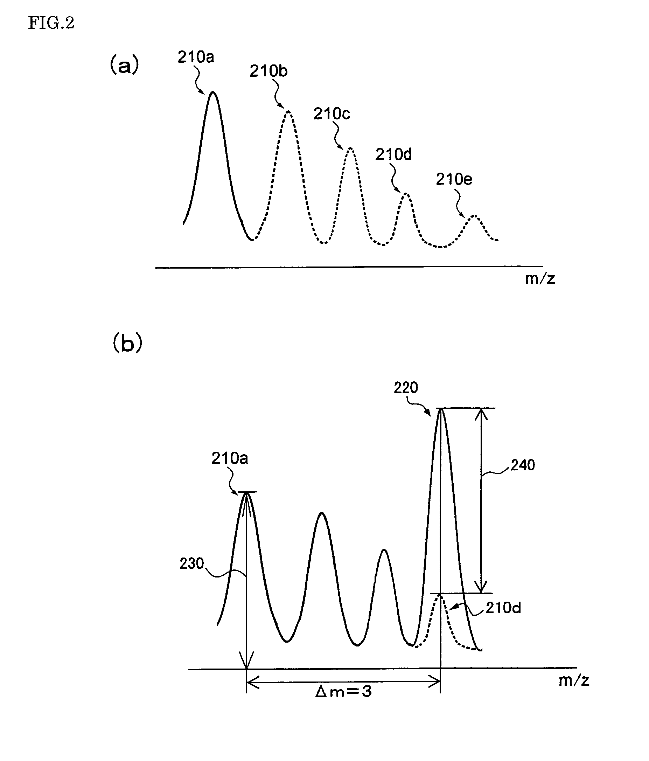

Fig 2 is a diagram showing the above described process. As shown in Fig 2(a), the peak of one peptide (symbol 210a) in the MS spectrum is accompanied by the peaks of isotopes which respectively exist in nature (symbols 210b, 210c, 210d, 210e...). In Fig 2(a), the peak having the lowest mass number from among these peaks is shown by a solid line, and others are shown by dotted lines.

Meanwhile, two types of samples A and B containing proteins are respectivel modified with labeling compounds O-methyl-isourea having different mass numbers, and therefore, in the MS spectrum of the mixed sample, the peak of the peptide that is labeled with a labeling compound having a heavy isotope (symbol 220) is located at such a distance that the mass number is 3 from the peak of the peptide that is labeled with a labeling compound having a light isotope (symbol 210a). Therefore, one of the peaks of the naturally-occurring isotopes which accompany the peptide that is labeled with a light labeling compound (symbol 210d in Fig 2(b)) overlaps with the peak of the peptide that is labeled with a heavy labeling compound (symbol 220). Thus, the height (symbol 240) of the peak which is obtained by subtracting the peak of symbol 210d from the peak of symbol 220 and the height (symbol 230) of the peak of symbol 210a are compared, and thereby, the mass ratio of the peptides which are represented by the respective peaks can be determined.

Here, though a case where the peak having the smallest mass number from among the peaks is used as a reference is shown, another peak, for example, the highest peak, may be used as a reference. In addition, analysis may, of course, be carried out using the peak areas.

Next, the amino acid sequence of each peptide is determined from the MS/MS spectrum. Here, for which peptide the amino acid sequence is identified can be selected on the basis of information on the above described MS spectrum. This selection may correspond to the purpose of analysis. In the case where only the portion that is different between sample A and sample B is desired to be analyzed, for example, it is possible to carry out analysis on only the peptide of which the content is different between sample A and sample B. Analysis may, of course, be carried out on peptides having the same content, or analysis may be carried out on all of the peptides. In this manner, which peptide should be analyzed can be selected, and therefore, samples can be analyzed efficiently.

When the amino acid sequence of a peptide is found as described above, this amino acid sequence and gene information on known proteins are compared using known software for retrieving data from a database where known DNA sequences are recorded (for example, Mascot (made by Matrix Science Ltd.) or the like), and thus, the protein which corresponds to the targeted peptide can be identified.

The ratio of the content of each peptide in sample A to that in sample B is found from the MS spectrum as described above, and therefore, the ratio of the content of a protein is found as the ratio of the content of the peptide which corresponds to this protein.

As described above, according to the method for analyzing proteins of the present embodiment, proteins which are included in two samples A and B can be identified from the MS/MS spectrum, and at the same time, the relative amount thereof can be found from the MS spectrum.

## Claims

1. A method for analyzing proteins according to which two types of samples containing proteins are compared using a mass spectrometer, so that the proteins which are included in respective samples are identified and the mass ratio of a protein of the same type that is included in the respective samples is analyzed, wherein the method for analyzing proteins is **characterized by** including the steps of:
respectively digestting said two types of samples containing proteins at portions of a certain amino acid using a restriction enzyme so as to prepare samples containing peptides;
modifying peptides which are included in said respective samples containing peptides with labeling compounds having different masses due to isotopes, so that peptides of the same type that are included in the respective samples containing peptides have different masses;
mixing the samples containing peptides that have been respectively labeled with isotopes, separating and quantifying the mixed sample for each peptide and measuring the MS spectrum, and finding the content ratio of peptides of the same type having different masses due to isotope labeling;
selecting a peptide of which the amino acid sequence should be identified from among the peptides in reference to said MS spectrum and qualitatively analyzing the amino acid sequence of selected peptide from the mass spectrum of the product ions which are generated from the peptide;
identifying a corresponding protein from known-DNA sequences on the basis of the amino acid sequence of said peptide; and
finding the ratio of the content of said identified protein included in said samples containing respective proteins on the basis of the value obtained from separation quantification using the difference in the mass of said peptides that have been modified with isotopes.

2. The method for analyzing proteins according to Claim 1, **characterized in that**
O-methyl-isourea and its stable isotopes are used as said labeling compounds.

3. The method for analyzing proteins according to Claim 2, **characterized in that**
in said step of finding the content ratio of peptides of the same type, when two peaks of peptides of the same type having different masses due to said modifying compounds in the MS spectrum are compared, the quantitative ratio is corrected by getting rid of the overlapping region with the peak of a peptide labeled with an naturally-occurring isotope.
